# EUROPEAN PATENT APPLICATION

(11) **EP 3 103 430 A1**
(43) Date of publication of application: **14.12.2016**
(21) Application number: 16748661.2
(22) Date of filing: 02.02.2016
(51) Int. Cl.: A61J 15/00, A61B 5/00

(54) **GASTRIC TUBE**

(30) Priority: 13.02.2015 CN 201510083548
(71) Applicant: Guangzhou General Hospital of Guangzhou Military Command, Guangzhou, Guangdong 510010 (CN)
(72) Inventor: ZHANG, Yijun, Guangzhou Guangdong 510010 (CN); ZHONG, Wuzhuang, Guangzhou Guangdong 510010 (CN); LIU, Jian, Guangzhou Guangdong 510010 (CN)
(74) Representative: Michalski Hüttermann & Partner Patentanwälte mbB
(86) International application number: PCT/CN2016/073159
(87) International publication number: WO 2016/127860

(57) **Abstract**

The present invention discloses a stomach tube for preventing aspiration, comprising: a control circuit, an air pump, a PH sensor and a stomach tube provided with an air sack, wherein the air pump is in communication with the air sack, the PH sensor is arranged on an inner wall of the stomach tube at a distance of 40cm away from a foretooth, and the control circuit is connected to the air pump and the PH sensor, respectively. The stomach tube of the present invention uses the PH sensor and the pressure sensor to monitor the PH value of the esophagus and the pressure of the air sack in real time, so as to accurately detect the occurrence of stomach esophagus regurgitation each time in advance, thereby avoiding the possibility of aspiration caused by regurgitation and the recessive pneumonia caused thereby; meanwhile, the present invention adopts a real-time control manner to automatically start the expansion of the air sack, thereby avoiding the damage to the esophageal mucosa caused by the long-time compression of the air sack on the esophagus, and reducing the substernal discomfort of a user. As a stomach tube for preventing aspiration, the present invention can be widely applied to the field of medical instruments.

## Description

### FIELD OF THE INVENTION

The present invention relates to the field of medical instruments, and particularly to a stomach tube for preventing aspiration.

### BACKGROUND OF THE INVENTION

A nasal feeding patient eats through an indwelled stomach tube. However, the probability of stomach esophagus regurgitation or aspiration pneumonia occurring in the patient who eats depending on nasal feeding is very high.

It is known to all that nasal feeding is for patients who cannot eat through mouth or have swallowing disorder, such as coma patients, patients with craniocerebral injury, patients with Parkinson's diseases, patients underwent gastrointestinal surgery or head and neck surgery, and critical patients. Performing enteral nutrition support through nasal feeding is one of the treatment means for emergent and critical patients, which can not only supply all nutrition that the bodies need and maintain energy and balance between water-salt and electrolyte, but also can keep normal physiological functions of the gastrointestinal tract to promote the early recovery of gastrointestinal functions, thereby being more economic and convenient as compared with parenteral nutrition. Moreover, the purpose of treating diseases can be achieved by injecting liquid medicine through a nasal feeding tube. However, long-time indwelling of the stomach tube will increase the risk of stomach esophagus regurgitation, which will result in regurgitation esophagitis and aspiration pneumonia, and the incidence rate is up to 10-22%. Meanwhile, a large amount of regurgitation in one time will result in death of patients from asphyxiation, and seriously endanger the health of patients. Therefore, it has great clinical significance if stomach esophagus regurgitation associated with the stomach tube can be reduced. However, the problem is also a difficult problem bothering most patients for a long time, and is also a hot research topic for medical workers.

Studies have reported that stomach esophagus regurgitation of some patients may be reduced by adjusting the intubation depth of the stomach tube, changing the diameter of the stomach tube or controlling the speed for food to flow into the stomach, adjusting the food capacity or raising the patient's head during feeding or by taking other measures, but still cannot prevent the stomach esophagus regurgitation of most patients. In recent years, domestic and foreign scholars have suggested that the probability of stomach esophagus regurgitation occurring in a patient will be greatly increased if the patient needs an indwelled stomach tube for more than one month. Therefore, gastrostomy is required to provide enteral nutrition, which has the purpose of reducing the occurrence rate of aspiration of patients, and is an expedient for reducing aspiration pneumonia associated with the stomach tube. However, as a creative operation, gastrostomy still cannot be accepted by most patients and family members thereof. Moreover, for patients with serious heart and lung diseases who cannot cooperate to perform gastroscopy examination or whose stomach wall and abdomen wall cannot get close to each other, for patients with seroperitoneum, peritonitis or diabetes or serious condition, and for some other patients, gastrostomy cannot be performed in primary hospitals. For some patients underwent gastrostomy, because exudation repeatedly occurs around the esophagus fistula and even food repeatedly back flows out from the esophagus fistula after eating, the feeding of patients is affected. Therefore, it has not been widely applied.

Comparatively speaking, as a simple routine operation in nursing, indwelling stomach tube can be widely applied to ordinary departments, and can be widely accepted by patients and family members thereof as well. Therefore, the prior art focuses on the development and research of a stomach tube for preventing aspiration.

Recently, scholars have improved the stomach tube in structure to prevent the occurrence of regurgitation. There are two novel stomach tubes for preventing aspiration in China, and both of the two stomach tubes are enlightened and improved on the basis of a three-cavity double-sack tube. One is a stomach tube (200620048639.8) for preventing aspiration researched and developed by Jiangsu Rugao Municipal People's Hospital in 2007. This type of stomach tube is a medical silica gel stomach tube, a first low-pressure cuff and a second low-pressure cuff are arranged on a front-end outer wall thereof, two air inflation catheters are arranged at the other end of the stomach tube, each of the air inflation catheters is provided with a first air inflation piston and a second air inflation piston, the first air inflation piston is in communication with the first low-pressure cuff by one air inflation catheter, the second air inflation piston is in communication with the second low-pressure cuff by the other air inflation catheter, and a gap between an outer wall of the stomach tube and an inner wall of the esophagus is blocked to prevent aspiration by simultaneously or alternately inflating the two low-pressure cuffs. Although the improved stomach tube reduces aspiration, it requires to alternately inflate air sacks to realize prevention of aspiration. However, the air sacks are fixed in position, so that the mucosa in the oppressed region of the esophagus is unavoidably damaged for a long time, and even esophageal ulceration or perforation is formed. Moreover, for patients with poor stomach dynamics, they will face the risk of regurgitation after 1-2 hours of injection of food. If there is a large amount of regurgitation, an air inflation device cannot be started to prevent the occurrence of aspiration caused by regurgitation.

The other stomach tube is a double-cavity air-sack stomach tube jointly developed by Shenzhen Luohu District People's Hospital and ZhanJiang Star Enterprise Co., Ltd, Guangdong in March 2007. Namely, the three-cavity double-sack tube frequently used in clinical is used as a major structure in which the esophagus sack is removed, and stomach sack and side holes are reserved to manufacture the dual-cavity air-sack stomach tube. 80-100ml of air is filled to an inlet of the air sack before injection of food, the stomach tube is outwards pulled and then fixed after there is resistance, and then food is injected into the stomach tube for 10-30 minutes, and the air sack is completely deflated after 60 minutes. However, using this type of stomach tube also has the same disadvantage as that of the previous stomach tube. It is also difficult to predict the time when regurgitation occurs in patients, so that handling cannot be performed in real time as well, thereby causing the occurrence of aspiration.

Foreign scholars mainly adopt the gastrostomy method to solve the nutrition problem of patients with stomach tubes indwelled for a long time, thereby reducing the occurrence of stomach esophagus regurgitation. However, there are still a considerable number of patients and some patients without indications cannot adopt the method. By consulting foreign literatures published recently, this problem in clinical is solved by reducing the diameter or length and material of the stomach tube. For patients having narrow esophagus caused by benign or malignant lesions of esophagus and having difficulty in swallowing, a method of implanting stents in the esophagus is employed to guarantee the nutrition supply of the patients.

To sum up, the prior art has the disadvantages that: 1. aspiration cannot be completely prevented; and 2. the device for preventing aspiration easily damages esophageal mucosa because of repeatedly oppressing the esophagus for a long time.

### SUMMARY OF THE INVENTION

To solve the above-mentioned technical problems, the object of the present invention is to provide a stomach tube for preventing aspiration and avoiding damaging esophageal mucosa for nasal feeding patients.

It is found through research that the PH value of the esophagus is less than 4 during stomach esophagus regurgitation. Therefore, in the present invention, a PH sensor is used to monitor the PH value of the esophagus in real time, and in case it is found by monitoring that the PH value of the esophagus is less than 4, the air pump is started to enable the air sack to expand so as to play the role of monitoring in real time and preventing regurgitation.

The present invention adopts the technical solution that: a stomach tube for preventing aspiration comprises a control circuit, an air pump, a PH sensor and a stomach tube provided with an air sack, wherein the air pump is in communication with the air sack, the PH sensor is arranged on an inner wall of the stomach tube at a distance of 40cm away from a foretooth, and the control circuit is connected to the air pump and the PH sensor, respectively.

Further, the control circuit comprises a PH comparison module, wherein the PH sensor is connected to an input end of the PH comparison module, and an output end of the PH comparison module is connected to the air pump.

Further, the stomach tube is provided with an air channel including an air port, and the air port is in communication with the air sack.

Further, an air inlet in communication with the air pump is arranged at an upper end of the air channel.

Further, a plug is arranged at a lower end of the air channel.

Further, the stomach tube is provided with a sensor line, and the control circuit is connected to the PH sensor by the sensor line.

Further, the sensor line is externally provided with a protective layer.

Further, a sealing hoop is externally arranged at the joint of the air sack and the stomach tube, and a liner ring is arranged inside the stomach tube at the joint of the air sack and the stomach tube.

Further, the stomach tube is made of a polyester soft material.

Further, a pressure sensor is also arranged in the air sack. The control circuit also comprises a pressure comparison module, wherein the pressure sensor is connected to an input end of the pressure comparison module, and an output end of the pressure comparison module is connected to the air pump.

The present invention has the beneficial effect that: the stomach tube of the present invention uses the PH sensor and the pressure sensor to monitor the PH value of the esophagus and the pressure of the air sack in real time, so as to accurately detect the occurrence of stomach esophagus regurgitation each time in advance, thereby avoiding the possibility of aspiration caused by regurgitation and the recessive pneumonia caused thereby; meanwhile, the present invention adopts a real-time control manner to automatically start the expansion of the air sack, thereby avoiding the damage to the esophageal mucosa caused by the long-time compression of the air sack on the esophagus, and reducing the substernal discomfort of a user.

### BRIEF DESCRIPTION OF THE DRAWINGS

Figure 1 is a block diagram of a structure of the present invention;
Figure 2 is a schematic diagram of a structure of a stomach tube of the present invention; and
Figure 3 is a schematic cross-sectional diagram of the structure of the stomach tube of the present invention.

In the figures: 1. air inlet; 2. stomach tube; 3. air channel; 4. nutrient solution channel; 5. sensor line; 6. air port; 7. stomach esophagus; 8. plug; 9. PH sensor; 10. sealing hoop; 11. air-sack tube; 12. air sack; 13. liner ring; 14. circuit interface.

### DETAILED DESCRIPTION OF THE EMBODIMENTS

The specific embodiments of the present invention will be further described below in conjunction with the accompanying drawings.

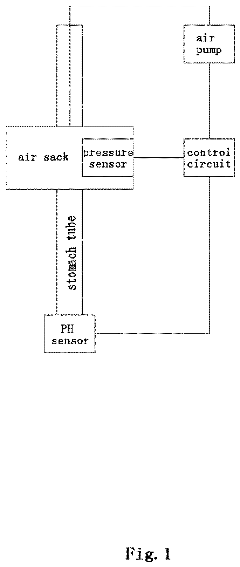

Referring to Figure 1, a stomach tube for preventing aspiration comprises a control circuit, an air pump, a PH sensor and a stomach tube provided with an air sack, wherein the air pump is in communication with the air sack, the PH sensor is arranged on an inner wall of the stomach tube at a distance of 40cm away from a foretooth, and the control circuit is connected to the air pump and the PH sensor, respectively.

Further, as a preferred embodiment, the control circuit comprises a PH comparison module, wherein the PH sensor is connected to an input end of the PH comparison module, and an output end of the PH comparison module is connected to the air pump.

The PH sensor of the present invention is connected to the air pump by the control circuit, and the air pump is started in case the PH value is less than 4, to enable the air sack to expand so as to prevent stomach esophagus regurgitation. On this basis, a control system of the stomach tube for preventing aspiration can be developed using the control circuit. The system operates according to set control parameters. It detects and controls the operating state and operating parameters of an air source, provides the operating state of a stomach acid sensor, forms a closed loop, and adjusts the output pressure of the air pump according to the PH detection result of the sensor in accordance with the developed control algorithm, to enable the stomach tube for preventing aspiration to always operate in the optimum state.

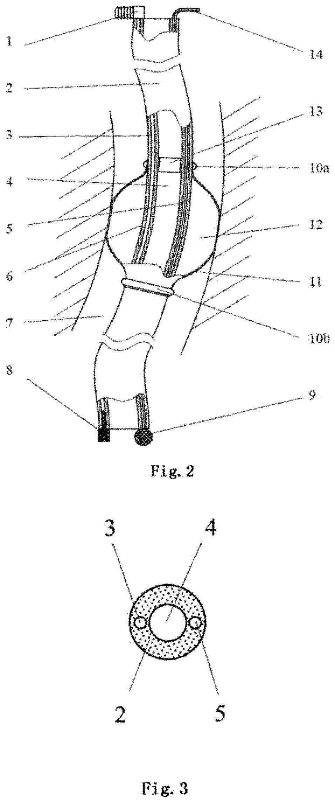

Referring to Figure 2, further, as a preferred embodiment, the stomach tube 2 is provided with an air channel 3 including an air port 6, and the air port 6 is in communication with the air sack 12.

Further, as a preferred embodiment, an air inlet 1 in communication with the air pump is arranged at an upper end of the air channel 3.

Further, as a preferred embodiment, a plug 8 is arranged at a lower end of the air channel 3. Further, as a preferred embodiment, the stomach tube 2 is provided with a sensor line 5, and the control circuit is connected to the PH sensor 9 by a circuit interface 14 on an upper part of the

Further, as a preferred embodiment, the sensor line 5 is externally provided with a protective layer.

The sensor line 5 and the air channel 3 are arranged at both sides of the tube wall of the stomach tube 2, as shown in Figure 3, and a nutrient solution channel 4 is formed in the middle of the stomach tube 3.

Further, as a preferred embodiment, a sealing hoop is externally arranged at the joint of the air sack 12 and the stomach tube 2, including an upper sealing hoop 10a and a lower sealing hoop 10b as shown in Figure 2, and a liner ring 13 is arranged inside the stomach tube at the joint of the air sack 12 and the stomach tube 2 (the liner ring corresponding to the lower sealing hoop 10b is not shown in Figure 2).

Further, as a preferred embodiment, the stomach tube is made of a polyester soft material. Referring to Figure 2, further, as a preferred embodiment, a pressure sensor is also arranged in the air sack. The control circuit also comprises a pressure comparison module, wherein the pressure sensor is connected to an input end of the pressure comparison module, and an output end of the pressure comparison module is connected to the air pump.

The pressure sensor is also connected to the control circuit by the sensor line.

According to data collected by the pressure sensor, a negative feedback loop of the pressure of the air sack is formed in the control system, to further strengthen accurate control on the pressure of the air sack.

Referring to Figure 2 and Figure 3, the operating flow of the present invention is explained.

First, this stomach tube is inserted into the stomach esophagus of a patient while in use. The cross section of the stomach tube 2 is provided with three hole channels, wherein the large hole 4 in the middle is used as a nutrient solution channel for importing nutrient solution required for patient's life. In addition, there are two small holes, wherein the hole 3 is used as an air channel, the compressed air of the air pump can pass through the air inlet 1 and enter the air sack 12 through the air channel 3 and the air hole 6, so that the air sack expands, and the stomach esophagus 7 is blocked by the air-sack tube 11. The other hole 5 in the cross section of the stomach tube is used as a sensor line channel, the line of the PH sensor 9 at the lowermost end of the stomach tube reaches the upper end of the stomach tube through the hole 5, and then is connected to the external circuit by the circuit interface 14.

The air-sack tube 11 and the stomach tube are fixed by the liner ring 13 embedded in the inner wall of the stomach tube and the outermost sealing hook 10.

The stomach tube 2 is a flexible tube with three through holes, an air outlet 6 is required to be machined in the middle to enable the compressed air to enter the air sack 12; meanwhile, a plug 8 is required to be additionally mounted at the bottom of the stomach tube 2, thereby preventing the compressed air from entering the human stomach.

The PH sensor 9 at the middle and lower part of the stomach tube 2 is used for detecting the PH value of the stomach juice of a patient. When the PH value is less than 4, the sampling voltage value acquired by the PH sensor 9 will be less than a set value of the PH comparison module in the control circuit. Therefore, the PH comparison module will output a high level such that the control system starts the air pump. Then, the compressed air is guided in the air sack 12, and the air sack 12 expands to block the stomach tube, so as to achieve the purpose of preventing stomach esophagus regurgitation.

When the pressure sensor detects that the pressure reaches the set value, the sampling voltage value acquired thereby will be greater than the set value of the pressure comparison module in the control circuit. Therefore, the pressure comparison module will output a low level such that the control system controls the air pump to stop operating. When the pressure is less than the set value, the air pump is automatically started, and the sack automatically expands, thereby guaranteeing that the air pressure is constant or changes in accordance with set requirements.

The invention is described in detail in accordance with the preferred embodiments. However, this invention is not limited to the embodiments. Those skilled in the art can make various equivalent modifications or replacements without departing from the spirit of the invention. All such equivalent modifications or replacements should fall within the scope defined by the claims of the invention.

## Claims

1. A stomach tube for preventing aspiration, **characterized by** comprising a control circuit, an air pump, a PH sensor and a stomach tube provided with an air sack, wherein the air pump is in communication with the air sack, the PH sensor is arranged on an inner wall of the stomach tube at a distance of 40cm away from a foretooth, and the control circuit is connected to the air pump and the PH sensor, respectively.

2. The stomach tube for preventing aspiration of claim 1, **characterized in that** the control circuit comprises a PH comparison module, wherein the PH sensor is connected to an input end of the PH comparison module, and an output end of the PH comparison module is connected to the air pump.

3. The stomach tube for preventing aspiration of claim 1, **characterized in that** the stomach tube is provided with an air channel including an air port, and the air port is in communication with the air sack.

4. The stomach tube for preventing aspiration of claim 3, **characterized in that** an air inlet in communication with the air pump is arranged at an upper end of the air channel.

5. The stomach tube for preventing aspiration of claim 3, **characterized in that** a plug is arranged at a lower end of the air channel.

6. The stomach tube for preventing aspiration of claim 1, **characterized in that** the stomach tube is provided with a sensor line, and the control circuit is connected to the PH sensor by the sensor line.

7. The stomach tube for preventing aspiration of claim 1, **characterized in that** the sensor line is externally provided with a protective layer.

8. The stomach tube for preventing aspiration of claim 1, **characterized in that** a sealing hoop is externally arranged at the joint of the air sack and the stomach tube, and a liner ring is arranged inside the stomach tube at the joint of the air sack and the stomach tube.

9. The stomach tube for preventing aspiration of claim 1, **characterized in that** the stomach tube is made of a polyester soft material.

10. The stomach tube for preventing aspiration of claim 1, **characterized in that** a pressure sensor is also arranged in the air sack, and the control circuit also comprises a pressure comparison module, wherein the pressure sensor is connected to an input end of the pressure comparison module, and an output end of the pressure comparison module is connected to the air pump.
